Europäisches Patentamt

European Patent Office

Office européen des brevets

(11) Numéro de publication: **0 261 736 B1**

(12) **FASCICULE DE BREVET EUROPEEN**

(19)

(45) Date de publication de fascicule du brevet: **14.04.93** (51) Int. Cl.⁵: **A61B 8/06**, G01S 15/89

(21) Numéro de dépôt: **87201800.7**

(22) Date de dépôt: **15.09.87**

(54) **Dispositif d'exploration par echographie ultrasonore d'un milieu en mouvement.**

(30) Priorité: **19.09.86 FR 8613140**

(43) Date de publication de la demande:
**30.03.88 Bulletin  88/13**

(45) Mention de la délivrance du brevet:
**14.04.93 Bulletin  93/15**

(84) Etats contractants désignés:
**BE DE ES FR GB IT NL SE**

(56) Documents cités:
**EP-A- 0 092 841**
**EP-A- 0 225 667**

(73) Titulaire: **LABORATOIRES D'ELECTRONIOUE PHILIPS**
**3, Avenue Descartes**
**F-94450 Limeil-Brévannes(FR)**

(84) Etats contractants désignés:
**FR**

(73) Titulaire: **N.V. Philips' Gloeilampenfabrieken**
**Groenewoudseweg 1**
**NL-5621 BA Eindhoven(NL)**

(84) Etats contractants désignés:
**BE DE ES GB IT NL SE**

(72) Inventeur: **Pesque, Patrick Société Civile S.P.I.D.**
**209, rue de l'Université**
**F-75007 Paris(FR)**

(74) Mandataire: **Charpail, François et al**
**Société Civile S.P.I.D. 156, Boulevard Haussmann**
**F-75008 Paris (FR)**

**Description**

La présente demande concerne un dispositif d'exploration par échographie ultrasonore d'un milieu en mouvement, permettant de mesurer la vitesse axiale dudit mouvement, soit la projection de la vitesse sur l'axe "z" d'un faisceau d'excitations ultrasonores périodiques émis, à une période de récurrence "T", par au moins un transducteur ultrasonore, comprenant, en outre, un étage d'émission, un étage de réception et de traitement des signaux échographiques renvoyés vers le transducteur.

L'objet de la présente demande trouve une application particulièrement avantageuse dans le domaine de l'exploration échographique d'organes en mouvement, tels que les parois du coeur, et d'écoulements sanguins.

Le problème technique à résoudre par tout procédé et tout dispositif d'exploration par échographie ultrasonore d'un milieu en mouvement, est d'obtenir une estimation aussi exacte que possible de la vitesse axiale du mouvement étudié afin de réaliser, à l'aide de dispositifs de visualisation, des images précises des organes et des écoulements sanguins soumis à une exploration échographique ultrasonore.

Depuis plusieurs années, diverses solutions à ce problème technique ont été proposées, en particulier les systèmes DOPPLER ultrasonores à onde pulsée, couramment utilisés pour mesurer en un point donné les vitesses d'écoulements sanguins, ou du moins la projection de telles vitesses sur l'axe du faisceau émis par les transducteurs ultrasonores. Plus récemment, sont apparus des appareils permettant d'obtenir en temps réel la distribution des vitesses d'écoulements sur le trajet parcouru par l'onde ultrasonore et, plus encore, sur le plan de coupe obtenu en balayant le transducteur. La plupart de ces systèmes exploitent le décalage de fréquence ou le décalage de phase du signal rétrodiffusé par les cibles mobiles pour en déduire la vitesse axiale des écoulements sanguins. La demande de brevet européen n° 0 092 841, par exemple, concerne un tel appareil utilisant la mesure du décalage de phase entre les échos successifs rétrodiffusés, en réponse à une excitation récurrente, par des cibles en mouvement.

Cependant, les appareils de mise en oeuvre de cette méthode connue utilisant le décalage de phase sont limités par une relation d'incertitude liant la résolution axiale $\Delta z$ et la précision de la mesure de vitesse $\Delta V/V$ à la longueur d'onde $\lambda$ :

$$\frac{\Delta V}{V} \cdot \Delta z = \frac{\lambda}{2}$$

Cette relation, citée dans le chapitre II, paragraphe 2.3-a, de l'ouvrage "Doppler Ultrasound and Its Use in Clinical Measurement", P. ATKINSON and J.P. WOODCOCK, Academic Press, 1982, impose donc un compromis entre la résolution axiale et la précision de la mesure de vitesse, ce qui est incompatible avec une mesure précise d'un profil de vitesse ou d'une image d'écoulements sanguins.

En ce sens, la demande de brevet européen EP-A- 0 225 667, qui n'est comprise dans l'état de la technique qu'au titre de l'Article 54(3) en (4) de la CBE, décrit un autre type de traitement du signal échographique dans lequel la précision de la mesure de la vitesse n'est pas limitée par la résolution spatiale. Il s'agit d'un procédé d'analyse temporelle qui consiste à interpréter la rétrodiffusion des signaux ultrasonores, non plus en termes de décalage de fréquence ou de phase, mais en termes de décalage temporel des signaux échographiques à la suite de chaque envoi de signaux impulsionnels. Ce procédé repose sur le principe suivant : soit un milieu en mouvement à la vitesse axiale $V_z(t)$ (la vitesse axiale est la projection de la vitesse sur l'axe "z" d'un faisceau d'excitations ultrasonores périodiques émis, à une période de récurrence "T", par un transducteur ultrasonore). Si à l'instant $t = 0$, alors que ledit milieu est à une distance "z" dudit transducteur, celui-ci émet une première excitation ultrasonore, l'écho $e_1(t)$ rétrodiffusé par le milieu sera reçu à l'instant $t_1 = 2z/C$, C étant la vitesse de propagation de l'onde ultrasonore. Puis, si à l'instant $t = T$, le transducteur émet une deuxième excitation ultrasonore, l'écho $e_2(t)$ renvoyé par le milieu en mouvement sera détecté par le transducteur à l'instant $t = T + 2[z + V_z(t)T]/C$, le milieu ayant parcouru la distance $V_z(t)T$ pendant la période "T". Prenant pour origine du temps pour le deuxième écho l'instant $t = T$ (origine de l'excitation correspondante), on obtient la relation $e_2(t) = e_1[t-\tau(t)]$ avec $\tau(t) = 2V_z(t)T/C$. Cette relation est très générale et peut s'écrire :

$$e_{i+1}(t) = e_i[t-\tau_i(t)] \qquad (1)$$

$$\tau_i(t) = 2V_{iz}(t)T/C \qquad (2)$$

$\tau_i$(t) étant alors le décalage temporel induit par le déplacement du milieu entre les tirs "i" et "i + 1".

On voit, par conséquent, sur la formule (2) que l'on peut mesurer la vitesse axiale $V_{iz}$(t) à partir du décalage temporel $\tau_i$(t), celui-ci étant extrait de la formule (1) par un traitement approprié.

La demande de brevet européen EP-A- 0 225 667 propose comme méthode d'extraction du décalage temporel $\tau_i$(t) l'usage des fonctions d'intercorrélation, le décalage temporel cherché étant celui pour lequel la fonction d'intercorrélation entre deux échos successifs $e_i$(t) et $e_{i+1}$(t) est maximale. Néanmoins, cette méthode, si elle a l'avantage de donner la vitesse axiale $V_{iz}$(t) de façon précise, présente l'inconvénient d'un dispositif de mise en oeuvre complexe exigeant, en plus d'un étage d'émission et d'un étage de réception et de traitement des signaux échographiques renvoyés vers le transducteur, de nombreux circuits corrélateurs, autant de circuits moyenneurs, et un circuit d'interpolation à microprocesseur ou à logique câblée.

Le problème technique général à résoudre par l'objet de la présente demande est de proposer un dispositif d'exploration par échographie ultrasonore d'un milieu en mouvement, comprenant, en outre, un étage d'émission, un étage de réception et de traitement des signaux échographiques renvoyés vers le transducteur, grâce auquel on obtient, à la fois, une mesure précise de la vitesse axiale sans limitation due à la résolution axiale, et une mise en oeuvre aisée à partir de circuits électroniques simples. Dans un mode particulier de réalisation relatif aux écoulements sanguins, il s'avère que le signal rétrodiffusé par le sang est très faible par rapport aux échos fixes. Le dispositif proposé devra alors permettre d'extraire le signal recherché malgré la présence de ces échos fixes parasites.

A cet effet le dispositif selon l'invention est caractérisé en ce que ledit étage de réception et de traitement des signaux échographiques comprend un circuit d'estimation de ladite vitesse axiale $V_{iz}$(t) comportant, d'une part, un circuit d'extraction du décalage temporel $\tau_i$(t) entre deux échos consécutifs $e_i$(t) et $e_{i+1}$(t), constitué pour résoudre l'équation en $\tau_i$(t) :

$$e_{i+1}(t) = \sum_{n=0}^{P} \frac{(-1)^n}{n!} \, e_i^{(n)}(t) . \tau_i^n(t) \qquad (3)$$

où $e_i^{(n)}$(t) est la dérivée d'ordre "n" par rapport au temps de l'écho $e_i$(t), et, d'autre part, un circuit de multiplication par C/2T de façon à retenir $V_{iz}$(t) à partir de $\tau_i$(t), C étant la vitesse de propagation de l'onde ultrasonore.

A titre d'exemple, on effectue ledit développement limité à l'ordre 1, le décalage temporel $\tau_i$(t) étant alors donné par :

$\tau_i$(t) = [$e_i$(t) - $e_{i+1}$(t)]/$e_i^{(1)}$(t)     (4)

On remarque sur cette formule que l'on peut ainsi déterminer le signe de $\tau_i$(t) et donc le signe de $V_{iz}$(t), ce qui permet d'obtenir le sens de la vitesse axiale. En effet, un décalage temporel négatif représente un mouvement se rapprochant du transducteur, tandis qu'à l'inverse, un décalage positif est caractéristique d'un mouvement s'éloignant du transducteur. Par ailleurs, le procédé lié à l'utilisation de la formule (4) peut être mis en oeuvre de façon très simplifiée à l'aide d'un dispositif tel que ledit circuit d'extraction du décalage temporel $\tau_i$(t) comporte une ligne à retard, un soustracteur, un circuit de calcul de dérivées premières par rapport au temps et un diviseur. Ce dispositif présente, en plus de sa simplicité, l'avantage de pouvoir être réalisé de façon entièrement analogique.

Dans le mode particulier de réalisation relatif aux écoulements sanguins, le circuit d'estimation de la vitesse axiale est précédé par un circuit d'élimination des échos fixes et d'atténuation des échos liés à des mouvements lents, comportant un soustracteur de deux lignes échographiques consécutives. Le signal résultant de cette différence est ensuite traité par le circuit d'estimation de la vitesse axiale. Comme on le verra plus loin en détail, la soustraction de deux lignes échographiques consécutives permet de s'affranchir des échos rigoureusement fixes et de limiter les effets des échos correspondant à des mouvements lents.

La description qui va suivre en regard des dessins annexés, donnés à titre d'exemples non limitatifs, fera bien comprendre en quoi consiste l'invention et comment elle peut être réalisée.

La figure 1 montre un exemple de réalisation d'un dispositif selon l'invention.

La figure 2 représente un mode d'exécution particulier de l'étage d'émission du dispositif de la figure 1.

La figure 3 est un mode de réalisation préférentiel d'un circuit d'estimation de la vitesse axiale.

La figure 4 montre un schéma d'un circuit de suppression des échos fixes et d'atténuation des échos liés à des mouvements lents.

La figure 5 est un diagramme donnant une loi de limitation des valeurs du décalage temporel $\tau_i(t)$.

La figure 6 représente un circuit discriminateur du dispositif de la figure 1.

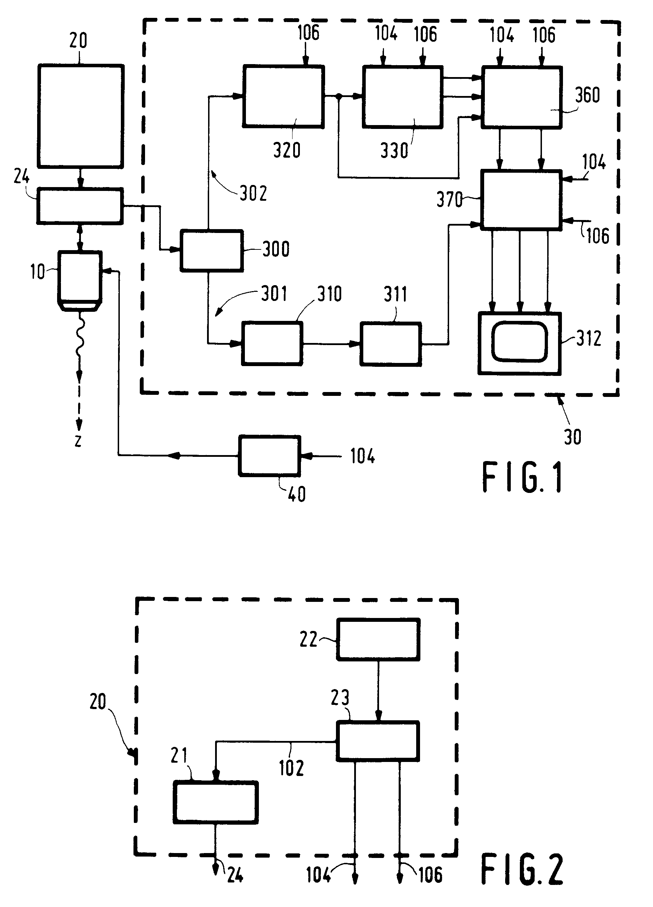

La figure 1 montre un schéma d'un dispositif d'exploration par échographie ultrasonore d'un milieu en mouvement, permettant de mesurer la vitesse axiale dudit mouvement, soit la projection de la vitesse sur l'axe "z" d'un faisceau d'excitations ultrasonores périodiques émis, à une période de récurrence "T", par un transducteur ultrasonore 10. Ce dispositif comprend, en outre, un étage d'émission 20, un étage 30 de réception et de traitement des signaux échographiques renvoyés vers le transducteur 10, ainsi qu'un dispositif 40 de commande de balayage mécanique du transducteur.

A la place de ce transducteur, pourrait bien entendu être utilisé un réseau de transducteurs, associé alors à un dispositif de commande de balayage électronique.

Dans la réalisation décrite de façon plus détaillée sur la figure 2, l'étage d'émission 20 comprend un générateur 21 de signaux électriques d'excitation, envoyés vers le transducteur 10 qui les convertit en trains périodiques de signaux impulsionnels ultrasonores. Cette émission est commandée par des signaux d'horloge disponibles sur une connexion 102 et délivrés à une fréquence de récurrence "F" -de l'ordre de 5 kilohertz par exemple- déterminée par un séquenceur comprenant successivement un oscillateur 22, ici de fréquence 32 mégahertz, et un diviseur de fréquence 23. Ce diviseur délivre les signaux d'horloge sur la connexion 102, ainsi que d'autres signaux de commande respectivement sur des connexions 104 et 106, à 1 kilohertz et 16 mégahertz dans l'exemple ici décrit. Les signaux de commande présents sur la connexion 104 commandent en particulier le dispositif 40, pour le balayage du transducteur. Un séparateur 24 des étages d'émission 20 et de réception et de traitement 30 est inséré entre le transducteur 10 et lesdits étages 20, 30 et évite l'aveuglement des circuits de réception par les signaux d'émission.

L'étage de réception et de traitement 30 comprend, en sortie du séparateur 24, un amplificateur haute fréquence 300 incluant la compensation de gain en fonction de la profondeur et suivi de deux voies de traitement 301 et 302 prévues en parallèle. La voie 301, de type traditionnel, comprend ici, en série, un détecteur d'enveloppe 310, un amplificateur de compression logarithmique 311, un dispositif de mémorisation et de conversion de balayage 370 incluant aussi une fonction de codage de couleur, et un dispositif 312 de visualisation. Cette voie 301 permet l'obtention, sous forme d'images en échelle de gris, de plans de coupe des milieux explorés selon le principe de l'échographie classique.

Comme on peut le voir à la figure 1, la deuxième voie 302 dudit étage 30 de réception et de traitement des signaux échographiques comprend un circuit 330 d'estimation de la vitesse axiale permettant, à partir d'une analyse temporelle du signal, d'extraire le décalage temporel $\tau_i(t)$ induit par le mouvement du milieu entre deux échos successifs $e_i(t)$ et $e_{i+1}(t)$ à l'aide d'un développement limité de la relation (1) :

$$e_{i+1}(t) = e_i[t - \tau_i(t)]$$

reliant les deux échos, soit :

$$e_{i+1}(t) = \sum_{n=0}^{P} \frac{(-1)^n}{n!} e_i^{(n)}(t) \cdot \tau_i^n(t) \qquad (3)$$

$e_{i+1}(t)$ pouvant être mesuré directement, et les dérivées $e_i^{(n)}(t)$ pouvant être calculées électroniquement. La relation (3) représente une équation en $\tau_i(t)$ qu'il suffit de résoudre pour en extraire le décalage temporel $\tau_i(t)$ cherché. La vitesse axiale $V_{iz}(t)$ s'en déduit par application de la formule suivante:

$$V_{iz}(t) = \tau_i(t)C/2T \qquad (5)$$

où C représente la vitesse de propagation de l'onde ultrasonore.

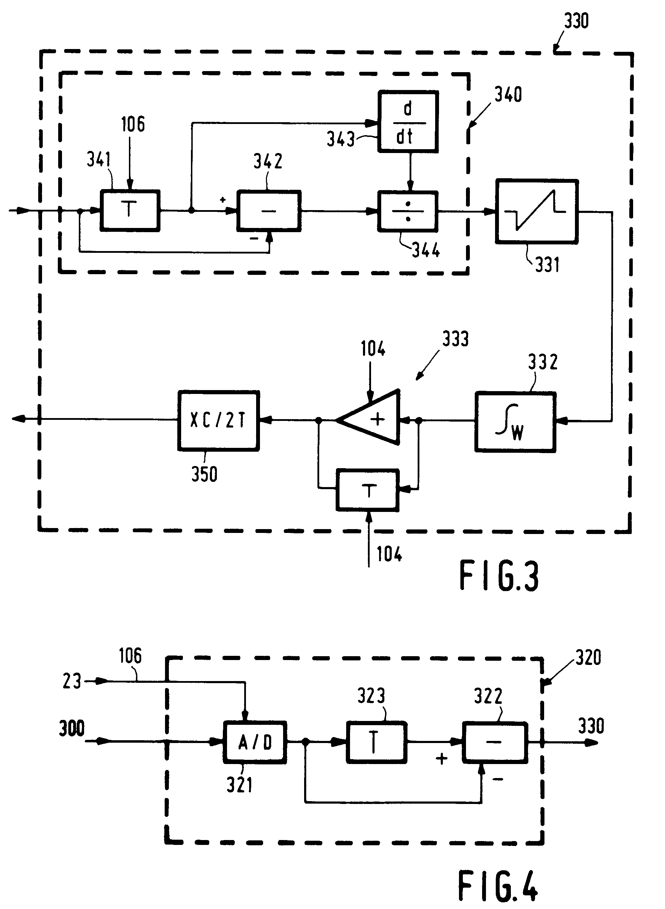

C'est pourquoi, conformément à la figure 3, le circuit 330 d'estimation de la vitesse axiale comporte, d'une part, un circuit 340 d'extraction du décalage temporel $\tau_i(t)$ constitué pour résoudre l'équation (3), et d'autre part, un circuit 350 de multiplication par C/2T.

En pratique, le décalage temporel $\tau_i(t)$ est très faible vis-à-vis des temps caractéristiques de variation du signal échographique. Par exemple, l'application de la formule (2) avec : T = 200 $\mu$s, C = 1 500 m/s et $V_{iz}(t)$ = 5 cm/s (écoulements sanguins, mouvements des parois du coeur) conduit à : $\tau_i(t)$ = 13,3 ns

Pour un signal échographique centré à 4 MHz, le décalage temporel est effectivement inférieur au dixième de la période du signal, soit 250 ns. En conséquence, un développement limité au premier ordre de la relation (1) est justifié. L'équation (3) s'écrit alors avec n = 1 :

$$e_{i+1}(t) = e_i(t) - e_i^{(1)}(t)\tau_i(t) \qquad (6)$$

Soit :

$$\tau_i(t) = [e_i(t) - e_{i+1}(t)]/e_i^{(1)}(t) \qquad (4)$$

Le circuit 340 d'extraction du décalage temporel, montré à la figure 3, représente un exemple, entièrement réalisé en analogique, de calcul de l'expression (6). A cet effet, le circuit 340 est composé d'une ligne à retard 341, d'un soustracteur 342, d'un circuit 343 de calcul de la dérivée première par rapport au temps et d'un diviseur 344. Une ligne à retard possédant les qualités requises : retard important (200 $\mu$s), très stable (250 ps) avec une grande dynamique (79 dB) et une bande passante élevée (4 MHz) est décrite dans FR-A-2 415 391.

Cependant, son implantation numérique est également concevable. Toutefois, le calcul de la dérivée nécessite quelques précautions. En effet, en échantillonnant le signal échographique au pas $\Delta T$ du signal 106 (60 ns dans l'exemple décrit), les points obtenus sont trop distants les uns des autres pour obtenir une estimée satisfaisante de la dérivée par la relation :

$$e_i^{(1)}(k\Delta T) = [e_i((k+1)\Delta T) - e_i(k\Delta T)]/\Delta T$$

La solution consiste à décaler, de façon analogique, l'écho $e_i(k\Delta T)$ d'un temps "$\epsilon$" petit devant $\Delta T$ ($\epsilon$ = 5 ns par exemple), puis à soustraire $e_i(k\Delta T-\epsilon)$ de $e_i k\Delta T$ et, enfin, à diviser la différence retenue par $\epsilon$, conformément à la formule :

$$e_i^{(1)}(k\Delta T) = [e_i(k\Delta T) - e_i(k\Delta T-\epsilon)]/\epsilon$$

En pratique, de façon à réduire le bruit d'estimation, il est avantageux de faire la moyenne du décalage temporel $\tau_i(t)$, calculé par la relation générale (3) et en particulier par la relation (4), sur une fenêtre temporelle de largeur W afin d'évaluer un décalage temporel moyen $\overline{\tau_i}(t)$ défini par :

$$\overline{\tau_i}(t) = \frac{1}{W} \int_t^{t+W} \tau_i(u)\,du$$

Cette opération est effectuée par un circuit intégrateur-moyenneur 332 situé entre le circuit 340 d'extraction du décalage temporel et le circuit 350 de multiplication par C/2T. On en déduit alors une vitesse axiale moyenne correspondante $V_{iz}(t)$ par :

$$\overline{V}_{iz}(t) = \overline{\tau_i}(t)C/2T$$

Après avoir calculé $\overline{\tau_i}(t)$ et avant multiplication par C/2T, on peut également faire une moyenne de $\tau_i(t)$ sur N tirs i consécutifs, à une cadence donnée par le signal 104, ce qui donne ici une moyenne sur N = 5 tirs. Cette moyenne est réalisée par le circuit 333 de la figure 3, en sortie de ce circuit on obtient donc :

$$\overline{\tau}(t) = \frac{1}{N} \sum_{k=1}^{N} \tau_k(t)$$

d'où la vitesse axiale :

$$\overline{V}_z(t) = \overline{\tau}(t)\, C/2T$$

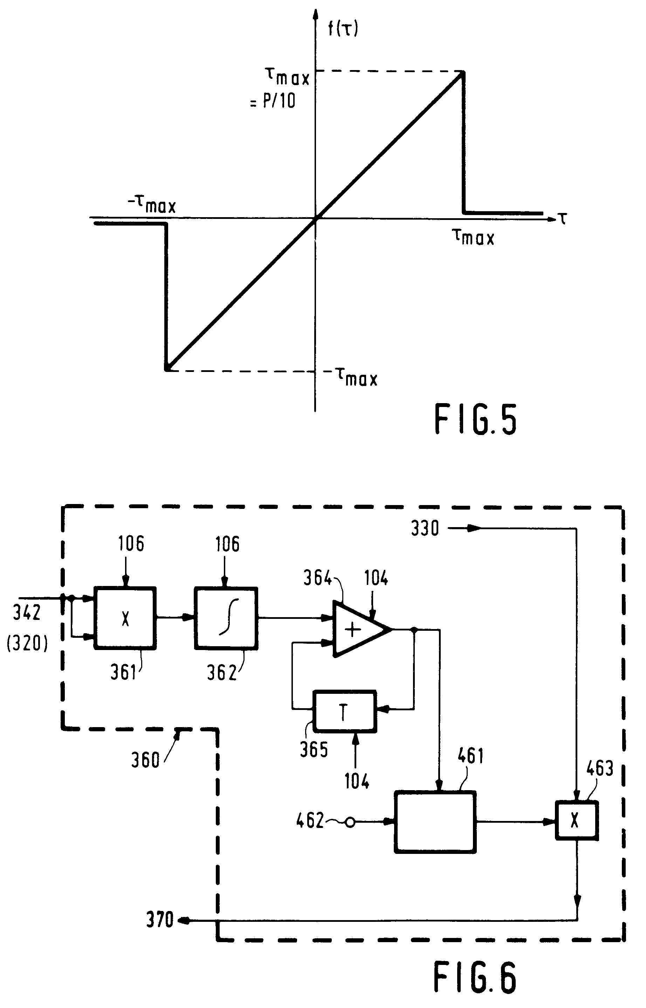

Par ailleurs, afin de rester dans le domaine de validité du développement limité défini par la relation (6) qui suppose $\tau_i(t)$ suffisamment petit, on a avantage à effectuer une limitation des valeurs de $\tau_i(t)$, représentée par une fonction de distribution $f(\tau)$ s'annulant lorsque $\tau$ dépasse en valeur absolue une valeur maximale $\tau_{max}$. La figure 5 montre un exemple d'une telle fonction de distribution : quand $\tau$ est compris

entre $-\tau_{max}$ et $\tau_{max}$, $f(\tau) = \tau$ et quand $|\tau| > \tau_{max}$, $f(\tau) = 0$. $\tau_{max}$ peut être pris égal au dixième de la période P du signal échographique soit 25 ns pour P = 250 ns. Dans ce cas, le décalage temporel moyen $\tau_i(t)$ est donné par :

$$\overline{\tau}_i(t) = \frac{1}{W} \int_t^{t+W} f[\tau_i(u)]du$$

Dans ce but, le circuit 330 d'estimation de la vitesse axiale comporte un circuit 331 de limitation des valeurs du décalage temporel, situé entre le circuit 340 d'extraction du décalage temporel et le circuit intégrateur-moyenneur 332.

Le signal de sortie du circuit 330 d'estimation de la vitesse axiale est alors validé ou non par un circuit discriminateur 360 montré à la figure 6, puis les valeurs ainsi confirmées sont envoyées vers le dispositif de visualisation 312 par l'intermédiaire du dispositif de codage de couleur 370.

La présence du circuit discriminateur 360 est indispensable. En effet, si les lignes échographiques successives, obtenues au rythme du déclenchement de l'excitation par le signal 102 à la fréquence F = 1/T, sont issues de cibles parfaitement fixes, le résultat de la différence entre ces deux lignes n'est que du bruit. Une ligne échographique s'écrit, d'une manière générale :

$y_i(t) = g(t) + e_i(t)$

où $g(t)$ est le signal dû aux cibles fixes et $e_i(t)$ l'écho produit par le milieu en mouvement.

La différence $d_i(t)$ entre deux lignes consécutives ves vaut donc :

$d_i(t) = y_{i+1}(t) - y_i(t) = e_{i+1}(t) - e_i(t)$     (7)

Si, pendant un intervalle de temps donné, les lignes échographiques $y_i(t)$ ne proviennent que de cibles fixes, on voit d'après (7) que $d_i(t) = 0$ au bruit près. Donc, le résultat fourni par le circuit 330 d'estimation de la vitesse axiale , qui traite ce bruit, n'est pas l'indication d'une vitesse nulle, et il devient nécessaire de valider ou non ce résultat. Le circuit 360 comprend, à cet effet, en série, un multiplieur 361 qui reçoit sur ces deux entrées le signal de sortie $d_i(t)$ du soustracteur 342 et élève ce signal de différence au carré. Un intégrateur 362 permet le calcul de l'énergie locale sur une fenêtre de largeur W′ (éventuellement égale à W) selon la formule :

$$E_i(t) = \int_t^{t+W'} d_i^2(u)du$$

Un circuit 364, 365 de calcul de moyenne qui, comme dans le cas du circuit 333 est un accumulateur comprenant un additionneur 364 et une ligne à retard 365 de retard T (ou un multiple de T), permet d'effecteur la moyenne de l'énergie locale sur M tirs c'est-à-dire (M - 1) différences selon l'expression :

$$E(t) = \frac{1}{M-1} \sum_{i=1}^{i=M-1} E_i(t) \qquad\qquad (12)$$

La valeur ainsi obtenue est envoyée vers un circuit de validation comprenant un comparateur 461 recevant d'une part, sur une première entrée, la sortie de l'accumulateur 364, 365, (ou directement celle du sommateur 362 dans le cas où le circuit de calcul de moyenne ne serait pas prévu) et d'autre part, sur une deuxième entrée 462, une tension de référence formant seuil. La sortie du comparateur est au niveau logique 0 ou 1 selon que la tension reçue sur sa première entrée est respectivement inférieure ou supérieure au seuil de référence $\alpha N(t)$ proportionnel au niveau de bruit $N(t)$. Un multiplieur 463, qui reçoit le signal de sortie $V_z(t)$ du circuit 330 sur une première entrée, transmet ce signal, noté désormais $V'_z(t)$, sur une sortie ou transmet simplement des valeurs nulles selon que le signal de validation délivré sur une deuxième entrée par le comparateur 461 est 1 ou 0 respectivement. En effet, en dehors des zones

6

d'écoulements vrais, l'énergie moyenne calculée en sortie du circuit 364, 365 est celle du bruit seul, et peut-être mesurée seule, a priori, en l'absence d'excitation, en vue de déterminer la valeur appropriée du seuil, c'est-à-dire que N(t) est également donné par :

$$\int_{t}^{t+W'} d_i^2(u)\,du$$

en dehors de toute excitation. Le niveau effectif du seuil est donc déterminé par le coefficient $\alpha$, au choix de l'utilisateur. Au contraire, en présence de signaux rétrodiffusés par les cibles en mouvement, l'énergie moyenne du signal $d_i(t)$ est supérieure à celle du bruit seul, ce qui autorise la validation des signaux délivrés par le circuit 330 d'estimation de la vitesse axiale.

Remarquons que la valeur de $\alpha$ peut servir aussi, à la visualisation, à fixer une frontière entre un affichage couleur et un affichage gris ; si E(t) est supérieur au seuil $\alpha$N(t), l'affichage sera en couleur. Par contre, il sera gris si E(t) est inférieur au seuil.

La sortie du circuit discriminateur 360 est envoyée vers le dispositif de mémorisation, conversion de balayage et codage de couleur 370, par lequel transite également, avant visualisation, le signal de sortie de l'amplificateur 311 de la voie de traitement 301. Un tel dispositif est décrit, par exemple, dans la demande de brevet européen EP-A-0 100 094. La figure 3 de ce document montre en effet, entre les bornes A, B, C, et $E_R$, $E_G$, $E_B$, un exemple des circuits qui peuvent être utilisés, la borne A recevant le signal échographique classique et les bornes B et C les paramètres caractéristiques du milieu en mouvement examiné. Ce dispositif 370 et le dispositif 312 permettent alors la visualisation en temps réel des écoulements ou des déplacements superposés à l'image de réflectivité échographique classique.

La figure 4 donne le schéma d'un circuit 320 de suppression des échos fixes et d'atténuation des échos liés à des mouvements lents, particulièrement nécessaire dans le cas d'étude d'écoulements sanguins.

Le circuit numérique 320 de suppression des échos, représenté sur la figure 4, comprend lui-même, dans cet exemple de réalisation, un convertisseur analogique-numérique 321 dont la sortie est reliée, d'une part, directement à l'entrée négative d'un soustracteur 322, et d'autre part, à l'entrée positive de ce soustracteur par l'intermédiaire d'un circuit à retard 323. Le retard apporté par ce dernier circuit 323 pourrait être de plusieurs périodes T = 1/F, mais il est avantageux que ce retard soit le plus faible possible et égal à T.

Le soustracteur 322 effectue donc la différence $d_i(t)$ entre deux lignes échographiques successives $y_i(t)$ et $y_{i+1}(t)$. C'est pourquoi, lorsque ce circuit 320 est présent, sa sortie peut être directement connectée à l'entrée du circuit discriminateur 360 qui exige aussi la connaissance de $d_i(t)$. D'autre part, la relation de récurrence :

$$e_{i+1}(t) = e_i[t - \tau_i(t)],$$

étant également vérifiée par $d_i(t)$, le signal de sortie du circuit 320 sert de signal d'entrée au circuit 330 d'estimation de la vitesse axiale.

Ce circuit 320 est prévu pour l'élimination de tous les échos fixes, en particulier de ceux dont l'apparition est provoquée par la réflexion des ondes ultrasonores contre les parois des vaisseux où se produisent les écoulements étudiés. Cette présence d'échos fixes est gênante du fait de leur amplitude bien plus élevée (de l'ordre de +40 dB dans le cas des écoulements sanguins) que celle des signaux utiles, les signaux rétrodiffusés par les cibles mobiles. Le circuit 320 est également commandé, par l'intermédiaire de la connexion 106, par le diviseur de fréquence 23 du séquenceur qui lui fournit le signal de commande d'échantillonnage à la fréquence de 16 MHz.

## Revendications

**1.** Dispositif d'exploration par échographie ultrasonore d'un milieu en mouvement, permettant de mesurer la vitesse axiale dudit mouvement, soit la projection de la vitesse sur l'axe "z" d'un faisceau d'excitations ultrasonores périodiques émis, à une période de récurrence "T", par au moins un transducteur ultrasonore (10), comprenant, en outre, un étage d'émission (20) et un étage (30) de réception et de traitement des signaux échographiques renvoyés vers le transducteur (10), caractérisé en ce que ledit étage (30) de réception et de traitement des signaux échographiques comprend un circuit (330) d'estimation de ladite vitesse axiale $V_{iz}(t)$ comportant, d'une part, un circuit (340) d'extraction du décalage temporel $\tau_i(t)$ entre deux échos successifs $e_i(t)$ et $e_{i+1}(t)$, constitué pour résoudre l'équation en $\tau_i(t)$ :

$$e_{i+1}(t) = \sum_{n=0}^{p} \frac{(-1)^n}{n!} e_i^{(n)}(t) . \tau_i^n(t)$$

où $e_i^{(n)}(t)$ est la dérivée d'ordre "n" par rapport au temps de l'écho $e_i(t)$, et, d'autre part, un circuit (350) de multiplication par C/2T de façon à obtenir $V_{iz}(t)$ à partir de $\tau_i(t)$, C étant la vitesse de propagation de l'onde ultrasonore.

2. Dispositif, selon la revendication 1, caractérisé en ce que, l'ordre "p" étant égal à 1, ledit circuit (340) d'extraction du décalage temporel $\tau_i(t)$ comporte une ligne à retard (341), un soustracteur (342), un circuit (343) de calcul de dérivée première par rapport au temps et un diviseur (344).

3. Dispositif, selon la revendication 2, caractérisé en ce que, le signal échographique étant numérisé par échantillonnage au pas $\Delta T$, le circuit de calcul de la dérivée première par rapport au temps comporte une ligne à retard décalant l'écho $e_i(k\Delta T)$ d'un temps $\epsilon$ petit devant $\Delta T$, un soustracteur réalisant la différence $e_i(k\Delta T) - e_i(k\Delta T - \epsilon)$, et un diviseur par $\epsilon$ de façon à obtenir la dérivée $e_i^{(1)}(k\Delta T)$.

4. Dispositif, selon l'une des revendications 1 à 3, caractérisé en ce que ledit circuit (330) d'estimation de la vitesse axiale $V_{iz}(t)$ comporte également un circuit intégrateur-moyenneur (332) de $\tau_i(t)$ sur une fenêtre de largeur W, situé entre le circuit (340) d'extraction du décalage temporel et le circuit (350) de multiplication par C/2T.

5. Dispositif, selon la revendication 4, caractérisé en ce que le circuit (330) d'estimation de la vitesse axiale $V_{iz}(t)$ comporte, en outre, un circuit (331) de limitation des valeurs du décalage temporel $\tau_i(t)$, situé entre le circuit (340) d'extraction du décalage temporel et le circuit intégrateur-moyenneur (332).

6. Dispositif, selon l'une des revendications 1 à 5, caractérisé en ce que le circuit d'estimation de la vitesse axiale est suivi d'un circuit discriminateur (360) comprenant un circuit (361) d'élévation au carré de la différence $d_i(t) = e_{i+1}(t) - e_i(t)$ entre deux échos successifs, un circuit sommateur (362) sur une fenêtre de largeur W′ donnant :

$$E(t) = \int_{t}^{t+W'} d_i^2(u) du$$

et un circuit de validation composé d'un comparateur de E(t) à un seuil $\alpha N(t)$ proportionnel au niveau de bruit N(t) et d'un multiplicateur (463) des valeurs de $V_{iz}(t)$ par 1 ou 0 suivant que E(t) est supérieur ou inférieur à $\alpha N(t)$.

7. Dispositif, selon l'une des revendications 1 à 6, caractérisé en ce que le circuit d'estimation (330) de la vitesse axiale est précédée par un circuit d'élimination (320) des échos fixes et d'atténuation des échos liés à des mouvements lents, comportant un soustracteur (322) de deux lignes échographiques consécutives.

## Claims

1. A device for examining a moving medium by means of ultrasonic echography, enabling the measurement of the axial speed of said movement, i.e. the projection of the speed on the axis "z" of a beam of ultrasound excitations which are periodically transmitted by at least one ultrasonic transducer (10) with a repetition period "T", which device also comprises a transmission stage (20) and a stage (30) for receiving and processing echographic signals returned to the transducer (10), characterized in that said stage (30) for receiving and processing echographic signals comprises a circuit (330) for estimating said axial speed $V_{iz}(t)$ which comprises, on the one hid a circuit (340) for extracting the time shift $\tau_i(t)$ between two successive echoes $e_i(t)$ and $e_{i+1}(t)$ which is constructed so as to solve the equation in $\tau_i(t)$:

$$e_{i+1}(t) = \sum_{n=0}^{p} \frac{(-1)}{n!} e_i^{(n)}(t) \cdot \tau_i^n(t)$$

where $e_i^{(n)}(t)$ is the derivative of the order "n" with respect to the time of the echo $e_i(t)$ and, on the other hand, a circuit (350) for multiplication by C/2T in order to obtain $V_{iz}(t)$ on the basis of $\tau_i(t)$, C being the propagation speed of the ultrasonic wave.

2.  A device as claimed in Claim 1, characterized in that, the order of the derivative "p" being equal to 1, said circuit (340) for extracting the time shift $\tau_i(t)$ comprises a delay line (341), a subtractor (342), a circuit (343) for calculating the first derivative with respect to time, and a divider (344).

3.  A device as claimed in Claim 2, characterized in that, the echographic signal being digitized by sampling with intervals $\Delta T$, the circuit for calculating the first derivative with respect to time comprises a delay line which shifts the echo $e_i(k\Delta T)$ by a period $\epsilon$ which is small with respect to $\Delta T$, a subtractor forming the difference $e_i(k\Delta T) - e_i(k\Delta T-\epsilon)$, id a divider by $\epsilon$ for obtaining the derivative $e_i^{(1)}(k\Delta T)$.

4.  A device as claimed in any one of the Claims 1 to 3, characterized in that said circuit (330) for estimating the axial speed $V_{iz}(t)$ also comprises an integrator/averaging circuit (332) for $\tau_i(t)$ on a window having a width W, situated between the circuit (340) for extracting the time shift and the circuit (350) for multiplication by C/2T.

5.  A device as claimed in Claim 4, characterized in that the circuit (330) for estimating the axial speed $V_{iz}(t)$ also comprises a circuit (331) for limiting the time shift values $\tau_i(t)$ which is situated between the circuit (340) for extracting the time shift and the integrator/averaging circuit (332).

6.  A device as claimed in any one of the Claims 1 to 5, characterized in that the circuit for estimating the axial speed is followed by a discriminator circuit (360) which comprises a circuit (361) for squaring the difference $d_i(t) = e_{i+1}(t) - e_i(t)$ between two successive echoes, a summing circuit (362) on a window having a width W' which provides:

$$E(t) = \int_t^{t+W'} d_i^2(u)\,du,$$

and a validation circuit which is composed of a comparator for comparing E(t) with a threshold $\alpha N(t)$ which is proportional to the noise level N(t) and a multiplier (463) for multiplying the values of $V_{iz}(t)$ by 1 or 0, depending on whether E(t) is greater or smaller than $\alpha N(t)$.

7.  A device as claimed in any one of the Claims 1 to 6, characterized in that the circuit (330) for estimating the axial speed is preceded by a circuit (320) for eliminating fixed echoes and for attenuating the echoes relating to slow motions, comprising a subtractor (322) for two consecutive echographic lines.

**Patentansprüche**

1.  Anordnung zum Untersuchen eines ortsveränderlichen Mediums durch Ultraschall-Echographie, die die Messung der axialen Geschwindigkeit der Bewegung ermöglicht, d.h. der Projektion der Geschwindigkeit auf der Achse "z" eines Bündels ausgestrahlter periodischer Ultraschallanregungen mit einer Wiederholungszeit "T" mit Hilfe mindestens eines Ultraschallwandlers (10), der außerdem eine Sendestufe (20) und eine Empfangs- und Bearbeitungsstufe (30) für die dem Wandler (10) zugesandten echographischen Signale enthält, dadurch gekennzeichnet, daß die Empfangs- und Bearbeitungsstufe (30) für die echographischen Signale eine Schaltung (330) zum Schätzen der axialen Geschwindigkeit

$V_{iz}(t)$ enthält, die einerseits eine Schaltung (340) zum Extrahieren der zeitbedingten Verschiebung $\tau_i(t)$ zwischen zwei aufeinanderfolgenden Echos $e_i(t)$ und $e_{i+1}(t)$ zum Lösen der Gleichung mit $\tau_i(t)$:

$$e_{i+1}(t) = \sum_{n=0}^{p} \frac{(-1)^n}{n!} e_i^{(n)}(t) \cdot \tau_i^n(t)$$

worin $e_i^{(n)}(t)$ die Ableitung in der Größenordnung "n" in bezug auf die Zeit des Echos $e_i(t)$ ist, und andererseits eine Schaltung (350) zum Multiplizieren mit C/2T enthält, um aus $\tau_i(t)$ $V_{iz}(t)$ zu erhalten, worin C die Fortpflanzungsgeschwindigkeit der Ultraschallwelle ist.

2. Anordnung nach Anspruch 1, dadurch gekennzeichnet, daß, wenn die Ordnung "p" gleich 1 ist, die Schaltung (340) zum Extrahieren der zeitbedingten Verschiebung $\tau_i(t)$ eine Verzögerungsleitung (341), einen Subtrahierer (342), eine Schaltung (343) zum Berechnen der ersten Ableitung gegen die Zeit und einen Teiler (344) enthält.

3. Anordnung nach Anspruch 2, dadurch gekennzeichnet, daß, wenn das echographische Signal durch Abtasten im Schritt $\Delta T$ digitalisiert wird, die Schaltung zum Berechnen der ersten Ableitung gegen die Zeit eine Verzögerungsleitung zum Verschieben des Echos $e_i(k\Delta T)$ um eine Zeit "$\epsilon$" kurz vor $\Delta T$, einen Subtrahierer zum Verwirklichen des Unterschieds $e_i(k\Delta T) - e_i(k\Delta T-\epsilon)$ und einen Teiler durch $\epsilon$ zum Erhalten der Ableitung $e_i^{(1)}(k\Delta T)$.

4. Anordnung nach einem der Ansprüche 1 bis 3, dadurch gekennzeichnet, daß die Schaltung (330) zum Schätzen der axialen Geschwindigkeit $V_{iz}(t)$ auch eine Integrator/Mittelwertbestimmungsschaltung (332) von $\tau_i(t)$ an einem Fenster der Größe W zwischen der Schaltung (340) zum Extrahieren der zeitbedingten Verschiebung und der Schaltung (350) zum Multiplizieren mit C/2T enthält.

5. Anordnung nach Anspruch 4, dadurch gekennzeichnet, daß die Schaltung (330) zum Schätzen der axialen Geschwindigkeit $V_{iz}(t)$ außerdem eine Schaltung (331) zum Begrenzen der zeitbedingten Verschiebungswerte $\tau_i(t)$ zwischen der Schaltung (340) zum Extrahieren der zeitbedingten Verschiebung und der Integrator/Mittelwertbestimmungschaltung (332) enthält.

6. Anordnung nach einem der Ansprüche 1 bis 5, dadurch gekennzeichnet, daß die Schaltung zum Schätzen der axialen Geschwindigkeit einer Diskriminatorschaltung (360) vorgeschaltet ist, die eine Schaltung (361) zum Quadraterheben des Unterschieds $d_i(t) = e_{i+1}(t) - e_i(t)$ zwischen zwei aufeinanderfolgenden Echos, eine Summierschaltung (362) an einem Fenster der Größe W' nach der Gleichung

$$E_i(t) = \int_t^{t+W'} d_i^2(u)\, du$$

und eine Auswerteschaltung enthält, die aus einem Komparator von E(t) bei einer Schwelle $\alpha N(t)$ proportional dem Geräuschpegel N(t) und einem Vervielfacher (463) der Werte von $V_{iz}(t)$ mit 1 oder 0 entsprechend E(t) höher oder niedriger als $\alpha N(t)$ besteht.

7. Anordnung nach einem der Ansprüche 1 bis 6, dadurch gekennzeichnet, daß die Schaltung (330) zum Schätzen der axialen Geschwindigkeit einer Schaltung (320) zum Unterdrücken von Festechos und zum Abschwächen von mit langsamen Bewegungen verknüpften Echos nachgeschaltet ist, die einen Subtrahierer (322) für zwei auffolgende echographische Linien enthält.

FIG.1

FIG.2

FIG.3

FIG.4

FIG.5

FIG.6